Europäisches Patentamt

European Patent Office

Office européen des brevets

(19)

(11) **EP 0 582 507 B1**

(12) **FASCICULE DE BREVET EUROPEEN**

(45) Date de publication et mention
de la délivrance du brevet:
**05.11.1997 Bulletin 1997/45**

(51) Int Cl.⁶: **E21B 37/06**, C10L 3/00,
C07C 7/20

(21) Numéro de dépôt: **93401960.5**

(22) Date de dépôt: **28.07.1993**

(54) **Méthode pour réduire la tendance à l'agglomération des hydrates dans des effluents de production**

Verfahren zur Reduktion der Agglomerationsneigung von Hydraten in Produktionsabströmungen

Process to reduce the tendency to agglomerate of hydrates in production effluents

(84) Etats contractants désignés:
**GB IT NL**

(30) Priorité: **03.08.1992 FR 9209686**

(43) Date de publication de la demande:
**09.02.1994 Bulletin 1994/06**

(73) Titulaire: **INSTITUT FRANCAIS DU PETROLE**
**92502 Rueil-Malmaison (FR)**

(72) Inventeurs:
• **Durand, Jean-Pierre**
  **F-78400 Chatou (FR)**
• **Baley, Anne-Sophie**
  **F-75006 Paris (FR)**
• **Gateau, Patrick**
  **F-78310 Maurepas (FR)**
• **Sugier, André**
  **F-92500 Rueil Malmaison (FR)**

(56) Documents cités:
**EP-A- 0 323 774**        **GB-A- 2 131 820**

Remarques:
Le dossier contient des informations techniques présentées postérieurement au dépôt de la demande et ne figurant pas dans le présent fascicule.

## Description

L' invention concerne un procédé pour réduire la tendance à l'agglomération des hydrates de gaz naturel, de gaz de pétrole ou d'autres gaz, par utilisation d'au moins un additif. Les gaz qui forment des hydrates peuvent notamment comprendre au moins un hydrocarbure choisi parmi le méthane, l'éthane, l'éthylène, le propane, le propène, le n-butane et l'iso-butane, et éventuellement de l'$H_2S$ et/ou du $CO_2$.

Ces hydrates se forment lorsque de l'eau se trouve en présence du gaz, soit à l'état libre, soit à l'état dissous dans une phase liquide, telle qu'un hydrocarbure liquide, et lorsque la température atteinte par le mélange notamment d'eau, de gaz et éventuellement d'hydrocarbures liquides, tels que de l'huile, devient inférieure à la température thermodynamique de formation des hydrates, cette température étant donnée pour une composition des gaz connue et lorsque leur pression est fixée.

La formation d'hydrates peut être redoutée, notamment dans l'industrie pétrolière et gazière, pour lesquelles les conditions de formation d'hydrates peuvent être réunies. En effet , pour diminuer le coût de production du pétrole brut et du gaz, tant au point de vue des investissements qu'au point de vue de l'exploitation, une voie envisagée, notamment en production en mer, est de réduire, voire de supprimer, les traitements appliqués au brut ou au gaz à transporter du gisement à la côte et notamment de laisser toute ou une partie de l'eau dans le fluide à transporter. Ces traitements en mer s'effectuent en général sur une plate-forme située en surface à proximité du gisement, de manière que l'effluent, initialement chaud, puisse être traité avant que les conditions thermodynamiques de formation des hydrates ne soient atteintes du fait du refroidissement de l'effluent avec l'eau de mer.

Cependant, comme cela arrive pratiquement, lorsque les conditions thermo-dynamiques requises pour former des hydrates sont réunies, l'agglomération des hydrates entraîne le remplissage et le blocage des conduites de transport par création de bouchons qui empêchent tout passage de pétrole brut ou de gaz.

La formation de bouchons d'hydrates peut entraîner un arrêt de la production et provoquer ainsi des pertes financières importantes. De plus, la remise en service de l'installation, surtout s'il s'agit de production ou de transport en mer, peut être ionique, car la décomposition des hydrates formés est très difficile à réaliser. En effet, lorsque la production d'un gisement sous-marin de gaz naturel ou de pétrole et de gaz comportant de l'eau atteint la surface du sol marin et est ensuite transportée au fond de la mer, il arrive par l'abaissement de la température de l'effluent produit,que les conditions thermo-dynamiques soient réunies pour que des hydrates se forment, s'agglomèrent et bloquent les conduites de transfert. La température au fond de la mer peut être, par exemple, de 3 ou 4°C.

Des conditions favorables à la formation d'hydrates peuvent aussi être réunies de la même façon à terre, pour des conduites pas ou trop faiblement enfouies dans le sol terrestre, lorsque par exemple la température de l'air ambiant est froide.

Pour éviter ces inconvénients, on a cherché, dans l'art antérieur, à utiliser des produits qui, ajoutés au fluide, pourraient agir comme inhibiteurs en abaissant la température thermodynamique de formation des hydrates. Ce sont notamment des alcools, tels que le méthanol, ou des glycols, tels que le mono-, le di- ou le triéthylèneglycol. Cette solution est très onéreuse car la quantité d'inhibiteurs à ajouter peut atteindre 10 à 30 % de la teneur en eau et ces inhibiteurs sont difficiles à récupérer complètement.

On a également préconisé l'isolation des conduits de transport, de manière à éviter que la température du fluide transporté n'atteigne la température de formation des hydrates dans les conditions opératoires. Une telle technique est, elle aussi, très coûteuse.

Par ailleurs, divers composés tensio-actifs non ioniques ou anioniques ont été testés pour leur effet de retardement de la formation d'hydrates au sein d'un fluide renfermant un gaz, notamment un hydrocarbure et de l'eau. On peut citer par exemple l'article de Kuliev et al : "Surfactants studied as hydrate - formation inhibitors" Gazovoe Delo n°10 1972, 17-19, rapporté dans Chemical Abstracts 80, 1974.98122 r.

On a encore décrit l'utilisation d'additifs capables de modifier le mécanisme de formation des hydrates, puisque au lieu de s'agglomérer rapidement les uns aux autres et de former des bouchons très solides, les hydrates formés se dispersent dans le fluide sans s'agglomérer et sans obstruer les conduites, tant que la température du fluide transporté n'est pas trop basse.

On peut citer, à cet égard : la demande de brevet EP-A-323774 au nom de la demanderesse, qui décrit l'utilisation de composés amphiphiles non-ioniques choisis parmi les esters de polyols et d'acides carboxyliques, substitués ou non-substitués, et les composés à fonction imide ; la demande de brevet EP-A-323775, également au nom de la demanderesse, qui décrit notamment l'utilisation de composés appartenant à la famille des diéthanolamides d'acides gras ou de dérivés d'acides gras ; le brevet US-A-4856593 qui décrit l'utilisation de composés tensio-actifs tels que des phosphonates organiques, des esters phosphates, des acides phosphoniques, leurs sels et leurs esters, des polyphosphates ioniques et leurs esters, ainsi que des polyacrylamides et des polyacrylates ; et la demande de brevet EP-A-457375, qui décrit l'utilisation de composé tensio-actifs anioniques, tels que les acides alkylarylsulfoniques et leurs sels de métaux alcalins.

On a maintenant découvert que des composés amphiphiles obtenus par réaction d'au moins un dérivé succinique choisi dans le groupe formé par les acides et les anhydrides polyalkénylsucciniques sur au moins un monoéther de polyéthylèneglycol pouvaient être avantageusement utilisés pour réduire la tendance à l'agglomération des hydrates de gaz naturel, de gaz de pétrole ou d'autres gaz.

Ainsi, l'invention propose un procédé pour réduire la tendance à l'agglomération des hydrates au sein d'un fluide comprenant de l'eau et un gaz, dans des conditions où des hydrates peuvent se former à partir d'eau et du gaz, caractérisé en ce qu'on incorpore audit fluide un additif comprenant au moins un composé amphiphile non-ionique obtenu par réaction d'au moins un dérivé succinique choisi dans le groupe formé par les acides et les anhydrides polyalkénylsucciniques sur au moins un éther de polyéthylèneglycol, la proportion molaire des réactifs étant de 0,5 à 2, de préférence voisine de 1.

Les dérivés succiniques utilisés pour préparer les composés utilisés dans l'invention ont habituellement une masse moléculaire moyenne en nombre d'environ 200 à 5000 et de préférence de 500 à 2000. Ces dérivés succiniques sont largement décrits dans l'art antérieur ; ils sont par exemple obtenus par l'action d'au moins une oléfine ou d'un hydrocarbure chloré sur l'acide ou l'anhydride maléïque. L'oléfine ou l'hydrocarbure chloré utilisé dans cette synthèse peuvent être linéaires ou ramifiés et comportent habituellement 10 à 200 atomes de carbone, de préférence 15 à 150 atomes de carbone et, le plus souvent, 20 à 100 atomes de carbone dans leur molécule. Cette oléfine peut également être un oligomère, par exemple un dimère, un trimère ou un tétramère, ou un polymère d'une oléfine inférieure, ayant par exemple 2 à 12 atomes de carbone, telle que l'éthylène, le propylène, le n-butène-1, l'isobutène, le n-hexène-1, le n-octène-1, le méthyl-2-heptène-1, le méthyl-2-propyl-5-hexène-1 ou le décène-1. L'hydrocarbure chloré peut provenir de la chloration de tels polymères. Il est possible d'utiliser des mélanges d'oléfines ou des mélanges d'hydrocarbures chlorés. Avantageusement, le dérivé succinique utilisé est un anhydride polyisobuténylsuccinique.

Les monoéthers de polyéthylèneglycol utilisés pour préparer les composés utlisés dans l'invention ont habituellement une masse moléculaire moyenne en nombre comprise entre 100 et 6000 et répondent à la fomule générale suivante

$$R\text{-}(\text{-O-CH}_2\text{-CH}_2)_n\text{OH} \qquad (1)$$

dans laquelle R est un groupe hydrocarboné renfermant par exemple de 1 à 30 atomes de carbone et n, représentant le degré moyen de polymérisation, à une valeur d'environ 2 à 140.

Les masses moléculaires respectives du dérivé succinique et du monoéther de polyéthylène-glycol considérés sont choisies de telle manière que le composé amphiphile résultant de leur réaction ait une valeur de HLB (de l'anglais "Hydrophilic Lipophilic Balance) au plus égale à 8 et de préférence comprise entre 2 et 7. La valeur du HLB peut être estimée selon la relation

$$HLB = \frac{E + C}{5}$$

où E représente le % masse d'oxyde d'éthylène dans la molécule et C représente le % masse de groupements carboxyles libres qui apportent également un caractère hydrophile à la molécule.

Dans la pratique, pour préparer les composés utilisables dans l'invention, on utilisera de préférence des monoéthers méthyliques de polyéthylèneglycol. La réaction entre le dérivé succinique et du monoéther de polyéthylèneglycol est conduite dans un solvant tel que le toluène, le xylène ou encore une coupe aromatique commerciale, à une température comprise par exemple entre 80 et 200°C.

Dans leur utilisation comme additifs pour réduire la tendance à l'agglomération des hydrates, ces composés sont ajoutés dans le fluide à traiter à des concentrations allant en général de 0,1 à 5 % en masse, de préférence de 0,25 à 2 % en masse, par rapport à l'eau.

Pour tester l'efficacité des produits utilisés dans le procédé de l'invention, on a simulé le transport de fluides formant des hydrates, tels que des effluents pétroliers et on a procédé à des essais de formation d'hydrates à partir de gaz, de condensat et d'eau, à l'aide de l'appareillage décrit ci-après.

L'appareillage comporte une boucle de 10 mètres constituée de tubes de diamètre intérieur égal à 7,7 mm ; un réacteur de 2 litres comprenant une entrée et une sortie pour le gaz, une aspiration et un refoulement pour le mélange : condensat, eau et additif initialement introduit. Le réacteur permet de mettre la boucle sous pression.

Des tubes de diamètre analogue à ceux de la boucle assurent la circulation des fluides de la boucle au réacteur, et inversement, par l'intermédiaire d'une pompe à engrenages placée entre les deux. Une cellule saphir intégrée dans le circuit permet une visualisation du liquide en circulation, et donc des hydrates, s'ils se sont formés.

Pour déterminer l'efficacité des additifs selon l'invention, on introduit les fluides (eau, condensat, additif) dans le réacteur ; l'installation est ensuite portée sous une pression de 70 bars. L'homogénéisation des liquides est assurée par leur circulation dans la boucle et le réacteur, puis uniquement dans la boucle. En suivant les variations de perte de charge et de débit, on impose une rapide diminution de la température, de 11°C à la température de formation des hydrates, celle-ci est ensuite maintenue à cette valeur.

La durée des tests peut varier de quelques minutes à plusieurs heures : un additif performant permet de maintenir la circulation de la suspension d'hydrates avec une perte de charge et un débit stables.

Les exemples suivants illustrent l'invention mais ne doivent en aucune manière être considérés comme limitatifs. L'exemple 3 est donné à titre comparatif.

## EXEMPLE 1

Dans un réacteur de 50 litres équipé d'un système d'agitation et d'un réfrigérant, on introduit 11,4 kg d'anhydride polyisobuténylsuccinique ayant un indice d'anhydride de 0,080 fonction anhydride/100 g, en solution dans 16,4 kg d'une coupe aromatique commerciale ayant une teneur en aromatiques de 99 %, un point initial de distillation de 286°C et un produit final de distillation de 214°C. Après l'addition de 5,0 kg de monométhyléther de polyéthylèneglycol dont la masse moléculaire moyenne est égale à 550, le mélange réactionnel obtenu est agité pendant 6 heures à 150-160°C, de façon à obtenir 32,8 kg d'une solution contenant 50 % en masse d'hémiester. Le produit a un HLB de 6,6.

## EXEMPLE 2

Dans le réacteur utilisé dans l'exemple 1, on introduit 12 kg d'anhydride polyisobuténylsuccinique ayant un indice d'anhydride de 0,080 fonction anhydride/- 100 g en solution dans 15,36 kg de xylène. Après l'addition de 3,36 kg de mono-méthyléther de polyéthylèneglycol dont la masse moléculaire moyenne est égal à 350, le mélange réactionnel ainsi obtenu est chauffé au reflux du xylène pendant 6 heures, conduisant à l'obtention de 30,72 kg d'une solution contenant 50 % en masse d'hémiester. Le produit a un HLB de 4,9.

## EXEMPLE 3 (comparatif)

Dans cet exemple, on opère avec un fluide composé en volume de 10 % d'eau et de 90 % de condensat. La composition pondérale du condensat est : pour les molécules ayant moins de 11 atomes de carbone : 20 % de paraffines et d'isoparaffines, 48 % de naphtènes, 10 % d'aromatiques ; et pour les molécules ayant au moins 11 atomes de carbone : 22 % d'un mélange de paraffines, d'isoparaffines, de naphtènes et d'aromatiques.

Le gaz utilisé comprend en volume 98 % de méthane et 2 % d'éthane. L'expérimentation est conduite sous une pression de 7 MPa, maintenue constante par apport de gaz. Dans ces conditions, on observe la formation d'un bouchon dans le serpentin, 10 minutes après le début de la formation des hydrates : les hydrates se sont formés en blocs.

## EXEMPLE 4

Dans cet exemple, on opère comme dans l'exemple 3 comparatif, avec le même fluide, le même gaz, et à la même pression, mais on ajoute au fluide en circulation 1 % en masse par rapport à l'eau, du produit fabriqué dans l'exemple 1. Dans ces conditions, on observe une augmentation de la perte de charge lors de la formation des hydrates à 4°C, suivie de sa diminution et de sa stabilisation pendant plus de vingt quatre heures. Une descente en température à 0°C n'affecte pas la circulation de la suspension, les hydrates restent dispersés dans les fluides.

## EXEMPLE 5

Toutes choses égales par ailleurs, on répète l'exemple 4 en utilisant 1 % en masse par rapport à l'eau du produit préparé comme décrit dans l'exemple 2. Dans ces conditions on observe, comme dans l'exemple 4, que la circulation du fluide est maintenue pendant plus de 24 heures.

## Revendications

1. Procédé pour réduire la tendance à l'agglomération des hydrates au sein d'un fluide comprenant de l'eau et un gaz, dans des conditions où des hydrates peuvent se former à partir d'eau et du gaz, caractérisé en ce qu'on incorpore audit fluide un additif comprenant au moins un composé amphiphile non-ionique obtenu par réaction d'au moins un dérivé succinique choisi dans le groupe formé par les acides et les anhydrides polyalkénylsucciniques sur au moins un monoéther de polyéthylèneglycol, la proportion molaire des réactifs étant de 0,5 à 2, ledit composé amphiphile ayant une valeur de HLB au plus égale à 8.

2. Procédé selon la revendication 1, caractérisé en ce que la proportion molaire des réactifs est voisine de 1.

3. Procédé selon la revendication 1 ou 2, caractérisé en ce que ledit dérivé succinique a une masse moléculaire moyenne en nombre d'environ 200 à 5000 et ledit monoéther de polyalkylèneglycol a une masse moléculaire moyenne en nombre de 100 à 6000 et répond à la formule générale :

$$R - (O\text{-}CH_2\text{-}CH_2\text{-})_n OH$$

dans laquelle R représente un groupe hydrocarboné de 1 à 30 atomes de carbone et r représentant le degré moyen de polymérisation a une valeur de 2 à 140.

4. Procédé selon l'une des revendications 1 à 3, caractérisé en ce que ledit composé amphiphile a une valeur de HLB comprise entre 2 et 7.

5. Procédé selon l'une des revendications 1 à 4, caractérisé en ce que ledit monoéther de polyéthylèneglycol est un monoéther méthylique de polyéthylèneglycol.

6. Procédé selon l'une des revendications 1 à 5, caractérisé en ce que le dérivé succinique est un anhydride polyisobuténylsuccinique de masse moléculaire moyenne en nombre de 500 à 2000.

7. Procédé selon l'une des revendications 5 et 6, caractérisé en ce que ledit monoéther méthylique de polyéthylèneglycol a une masse moléculaire moyenne de 550 et ledit anhydride polyisobutényl succinique renferme 0.080 fonction anhydride pour 100 grammes.

8. Procédé selon l'une des revendications 4 et 5, caractérisé en ce que ledit monoéther méthylique de polyéthylèneglycol a une masse moléculaire moyenne de 350 et ledit anhydride polyisobutényl succinique renferme 0,080 fonction anhydride pour 100 grammes.

9. Procédé selon l'une des revendications 1 à 8, caractérisé en ce que ledit composé amphiphile non-ionique est incorporé audit fluide a une concentration de 0,1 à 5% en masse par rapport à l'eau présente.

10. Procédé selon la revendication 9, caractérisé en ce que ladite concentration est de 0,25 à 2% en masse par rapport à l'eau présente.

11. Procédé selon l'une des revendications 1 à 10, caractérisé en ce que, dans ledit fluide, ledit gaz comprend au moins un hydrocarbure choisi parmi le méthane, l'ethane, l'éthylène, le propane, le propène, le n-butane, et l'iso-butane, et éventuellement de l'$H_2S$ et/ou du $CO_2$.

12. Procédé selon l'une des revendication 1 à 10, caractérisé en ce que ledit fluide comprend du gaz naturel.

13. Procédé selon l'une des revendication 1 à 10, caractérisé en ce que ledit fluide comprend du gaz de pétrole et au moins un hydrocarbure liquide.

14. Procédé selon l'une des revendications 1 à 10, caractérisé en ce que ledit fluide comprend de l'huile, du gaz et de l'eau.

**Patentansprüche**

1. Verfahren zur Verminderung der Neigung zur Verdichtung von Hydraten in Fluiden, welche Wasser und ein Gas umfassen, unter Bedingungen, in denen sich Hydrate aus Wasser und Gas bilden können, dadurch gekennzeichnet, daß man in das Fluid ein Additiv einbringt, das wenigstens eine nicht-ionische amphiphile Verbindung umfaßt, welche durch Umsetzung wenigstens eines Bernsteinsäurederivates, das aus der durch die Polyalkenylbernsteinsäuren und -anhydride gebildeten Gruppe ausgewählt ist, mit wenigstens einem Monoether von Polyethylenglycol erhält, wobei das molare Verhältnis der Reagenzien von 0,5 bis 2 beträgt und die amphiphile Verbindung einen HLB-Wert von höchstens 8 aufweist.

2. Verfahren nach Anspruch 1, dadurch gekennzeichnet, daß das molare Verhältnis der Reagenzien nahe 1 liegt.

3. Verfahren nach Anspruch 1 oder 2, dadurch gekennzeichnet, daß das Bernsteinsäurederivat eine zahlenmittlere Molekularmasse von etwa 200 bis 5.000 besitzt und der Polyalkylenglycolmonoether eine zahlenmittlere Molekularmasse von 100 bis 6.000 besitzt und der allgemeinen Formel:

$$R\text{-}(O\text{-}CH_2\text{-}CH_2\text{-})_n OH$$

entspricht, worin R eine Kohlenwasserstoffgruppe mit 1 bis 30 Kohlenstoffatomen bedeutet und n, das den mittleren Grad der Polymerisation darstellt, einen Wert von 2 bis 140 aufweist.

4. Verfahren nach einem der Ansprüche 1 bis 3, dadurch gekennzeichnet, daß die amphiphile Verbindung einen HLB-Wert aufweist, der zwischen 2 und 7 liegt.

5. Verfahren nach einem der Ansprüche 1 bis 4, dadurch gekennzeichnet, daß der Polyethylenglycolmonoether ein Methylmonoether von Polyethylenglycol ist.

6. Verfahren nach einem der Ansprüche 1 bis 5, dadurch gekennzeichnet, daß das Bernsteinsäurederivat ein Polyisobutenbernsteinsäureanhydrid mit einer zahlenmittleren Molekularmasse von 500 bis 2.000 ist.

7. Verfahren nach einem der Ansprüche 5 und 6, dadurch gekennzeichnet, daß der Methylmonoether von Polyethylenglycol eine mittlere Molekularmasse von 550 besitzt und das Polyisobutenylbernsteinsäureanhydrid 0,080 Anhydridfunktionen pro 100 g umfaßt.

8. Verfahren nach einem der Ansprüche 4 und 5, dadurch gekennzeichnet, daß der Methylmonoether von Polyethylenglycol eine mittlere Molekularmasse von 350 besitzt und das Polyisobutenylbernsteinsäureanhydrid 0,080 Anhydridfunktionen pro 100 g besitzt.

**9.** Verfahren nach einem der Ansprüche 1 bis 8, dadurch gekennzeichnet, daß die nicht-ionische amphiphile Verbindung in das Fluid in einer Konzentration von 0,1 bis 5 Masse-% bezogen auf anwesendes Wasser eingebracht wird.

**10.** Verfahren nach Anspruch 9, dadurch gekennzeichnet, daß die Konzentration 0,25 bis 2 Masse-% bezogen auf das anwesende Wasser beträgt.

**11.** Verfahren nach einem der Ansprüche 1 bis 10, dadurch gekennzeichnet, daß in dem Fluid das Gas wenigstens einen Kohlenwasserstoff, der aus Methan, Ethan, Ethylen, Propan, Propen, n-Butan und i-Butan ausgewählt ist, und gegebenenfalls $H_2S$ und/oder $CO_2$ umfaßt.

**12.** Verfahren nach einem der Ansprüche 1 bis 10, dadurch gekennzeichnet, daß das Fluid Erdgas umfaßt.

**13.** Verfahren nach einem der Ansprüche 1 bis 10, dadurch gekennzeichnet, daß das Fluid Erdölgas und wenigstens einen flüssigen Kohlenwasserstoff umfaßt.

**14.** Verfahren nach einem der Ansprüche 1 bis 10, dadurch gekennzeichnet, daß das Fluid, Öl, Gas und Wasser umfaßt.

## Claims

**1.** A process for reducing the agglomeration tendency of hydrates within a fluid containing water and a gas, under conditions where hydrates can form from the water and the gas, characterised in that incorporated into said fluid is an additive containing at least one non-ionic amphiphilic compound obtained by reacting at least one succinic derivative, chosen from the group formed by polyalkenylsuccinic acids and anhydrides, on at least one polyethylene glycol monoether, the molar proportion of reagents being 0.5 to 2, said amphiphilic compound having a hydrophilic lipophilic balance (HLB) value which is at the most equal to 8.

**2.** A process according to Claim 1, characterised in that the molar proportion of the reagents is close to 1.

**3.** A process according to Claim 1 or Claim 2, characterised in that said succinic derivative has a number average molecular weight of approximately 200 to 5000, and said polyalkylene glycol monoether has a number average molecular weight of 100 to 6000 and corresponds to the general formula:

$$R - (O\text{-}CH_2\text{-}CH_2\text{-})_n OH$$

in which R represents a hydrocarbon group having 1 to 30 carbon atoms and r represents the average degree of polymerization at a value of 2 to 140.

**4.** A process according to one of Claims 1 to 3, characterised in that said amphiphilic compound has a HLB value of between 2 and 7.

**5.** A process according to one of Claims 1 to 4, characterised in that said polyethylene glycol monoether is a polyethylene glycol methyl monoether.

**6.** A process according to one of Claims 1 to 5, characterised in that the succinic derivative is a polyisobutenylsuccinic anhydride with a number average molecular weight of 500 to 2000.

**7.** A process according to either Claim 5 or Claim 6, characterised in that said polyethylene glycol methyl monoether has an average molecular weight of 550, and said polyisobutenyl succinic anhydride contains 0.080 anhydride function per 100 grammes.

**8.** A process according to either Claim 4 or Claim 5, characterised in that said polyethylene glycol methyl monoether has an average molecular weight of 350, and said polyisobutenyl succinic anhydride contains 0.080 anhydride function per 100 grammes.

**9.** A process according to one of Claims 1 to 8, characterised in that said non-ionic amphiphilic compound is incorporated into said fluid at a concentration of 0.1 to 5% by weight, based on the water present.

**10.** A process according to Claim 9, characterised in that said concentration is 0.25 to 2% by weight, based on the water present.

**11.** A process according to one of Claims 1 to 10, characterised in that in said fluid said gas comprises at least one hydrocarbon selected from methane, ethane, ethylene, propane, propene, n-butane, and iso-butane, and possibly $H_2S$ and/or $CO_2$.

**12.** A process according to one of Claims 1 to 10, characterised in that said fluid comprises natural gas.

**13.** A process according to one of Claims 1 to 10, characterised in that said fluid comprises petroleum gas and at least one liquid hydrocarbon.

**14.** A process according to one of Claims 1 to 10, characterised in that said fluid comprises oil, gas and water.